(19)

Europäisches
Patentamt
European
Patent Office
Office européen
des brevets

(11) **EP 1 694 196 B1**

(12) **EUROPEAN PATENT SPECIFICATION**

(45) Date of publication and mention
of the grant of the patent:
**19.10.2011  Bulletin 2011/42**

(21) Application number: **03775695.4**

(22) Date of filing: **27.11.2003**

(51) Int Cl.:
***A61B 5/00*** (2006.01)

(86) International application number:
**PCT/IB2003/005704**

(87) International publication number:
**WO 2005/053526 (16.06.2005 Gazette 2005/24)**

(54) **TECHNIQUES FOR DETERMINING GLUCOSE LEVELS**

TECHNIKEN ZUR BESTIMMUNG DES GLUCOSEGEHALTES

TECHNIQUES POUR DETERMINER LE TAUX DE GLUCOSE

(84) Designated Contracting States:
**AT BE BG CH CY CZ DE DK EE ES FI FR GB GR
HU IE IT LI LU MC NL PT RO SE SI SK TR**

(43) Date of publication of application:
**30.08.2006  Bulletin 2006/35**

(60) Divisional application:
**10008904.4 / 2 277 438**

(73) Proprietor: **Solianis Holding AG
6300 Zug (CH)**

(72) Inventors:
• **CADUFF, Andreas
CH-8005 Zürich (CH)**

• **DEWARRAT, Rodolphe
CH-8032 Zürich (CH)**

(74) Representative: **Sutter, Kurt et al
E. Blum & Co. AG
Vorderberg 11
8044 Zürich (CH)**

(56) References cited:
**WO-A-02/069791          US-A- 5 050 612
US-A- 5 077 476          US-A1- 2002 106 709
US-A1- 2003 153 821     US-B1- 6 309 884**

EP 1 694 196 B1

**Description**

Technical Field

The invention relates to devices for the determination of glucose and to methods for measuring glucose.

Background Art

It has been known that the glucose of living tissue can be measured non-invasively by applying a sensor arrangement, in particular an electrode arrangement to the skin of a patient and measuring the response of the electrode arrangement to a suitable electric signal. Such a technique is described in WO 02/069791, the disclosure of which is enclosed herein in its entirety.

Even though this type of device is well able to monitor glucose, it needs careful calibration according to a well-defined protocol and must be operated under defined conditions in order to yield results of high accuracy.

One important purpose of these devices is to provide a prediction of the time when a patient's glucose level may exceed certain limits. In particular, an early prediction of a possible hypoglycemia or hyperglycemia is desirable such that a patient or accompanying person may take adequate steps to prevent such a state.

A device with this function is described in US 2002/0106709. It uses a Taylor series expansion to predict the glucose level from measured glucose values.

Disclosure of the Invention

The problem to be solved by the present invention is to provide a device and method that are capable to provide early and reliable prediction of a possible hyper- or hypoglycemia. This object is achieved by the device and method of the independent claims 1 and 25.

Advantageously, at least two temperatures are measured, wherein the first temperature depends in different manner on the skin temperature of the body and on the environmental temperature than the second temperature when the device is mounted on the body in its position of operation. This allows to compensate for the influence of both, the skin temperature and the environmental temperature, which has been found to be advantagous because, as discussed in the detailed description, both temperatures affect the signals measured with the sensor arrangement in different manners.

Advantageously, the object of the invention is achieved by improving the calibration of the device. Two different calibration mechanisms are suggested, which can be used alternatively or in combination.

In both these mechanisms, the device is assumed to calculate, in normal operation, the glucose level from a function of the type $F(s_1, s_2; ... s_N, a_0, a_1, ... a_M)$, where F depends on input values $s_1 ... s_N$ and calibration parameters $a_0 ... a_M$.

In a calibration phase, series of reference values $g(t_i)$ are obtained at times ti, e.g. using conventional glucose measurements. In the same phase, a series of raw input values $s_j(t'_i)$ are measured at times $t'_i$, which generally do not necessarily coincide with the times $t_i$. At least part of the parameters $a_0 ... a_M$ are then derived from these measurements by comparing the values obtained by function F against the reference values or against values derived from the reference values.

In most cases, the number of times the input values $s(t'_i)$ have been measured will be considerably larger than the number of reference values $g(t_i)$. Hence, in the first mechanism, in order to fully exploit all data, a prediction (interpolation) of the glucose level g at the times $t'_i$ is calculated from the reference values $g(t_i)$. Then the deviation of the values calculated by function F for the input values $s_j(t'_i)$ and the predicted glucose levels at times $t'_j$ is minimized by varying the parameters $a_0 ... a_M$, thereby obtaining a set of calibrated parameters.

In the second mechanism, a "shift correction" is carried out during the calibration phase. For this purpose, the times $\tau_i$ are detected at which the device has shifted or moved in relation to the body during calibration. Such shifts generally cause the measured signals to change. When comparing the values obtained by function F against the reference values or against values derived from the reference values as mentioned above, at least one parameter $a_0$ is replaced by a sum

$$\sum_{i=0}^{P} a_{0i} \cdot b_i(t)$$

with bi(t) being 1 (or, equivalently, any other non-zero constant value) for $\tau_i < t < \tau_{i+1}$ and 0 otherwise. As explained in the detailed description, this allows to compensate for the effects of the shifts.

In any case it may be advantageous to carry out a recalibration step, e.g. each time after putting on the device. In this step, one of the parameters is recalibrated to find an optimum agreement between the glucose level calculated from the function F and a glucose level from a reference measurement. The reference measurement can e.g. be a conventional measurement, such as an invasive measurement. This allows to compensate for an offset caused by removing and remounting the device.

The various aspects and mechanisms can be used in combination or separately.

Brief Description of the Drawings

The invention will be better understood and objects other than those set forth above will become apparent when consideration is given to the following detailed description thereof. Such description makes reference to the annexed drawings, wherein:

Fig. 1 is a cross section of a device for measuring a glucose level,
Fig. 2 is a block circuit diagram of the device of Fig. 1,
Fig. 3 is an apparatus for calibrating the device,
Fig. 4 illustrates an advantageous aspect of the calibration method,
Fig. 5 shows a signal shift upon a displacement of the device, and
Fig. 6 illustrates a worst-case prediction of glucose levels with and without limits for the second order derivative.

Modes for Carrying Out the Invention

The device:

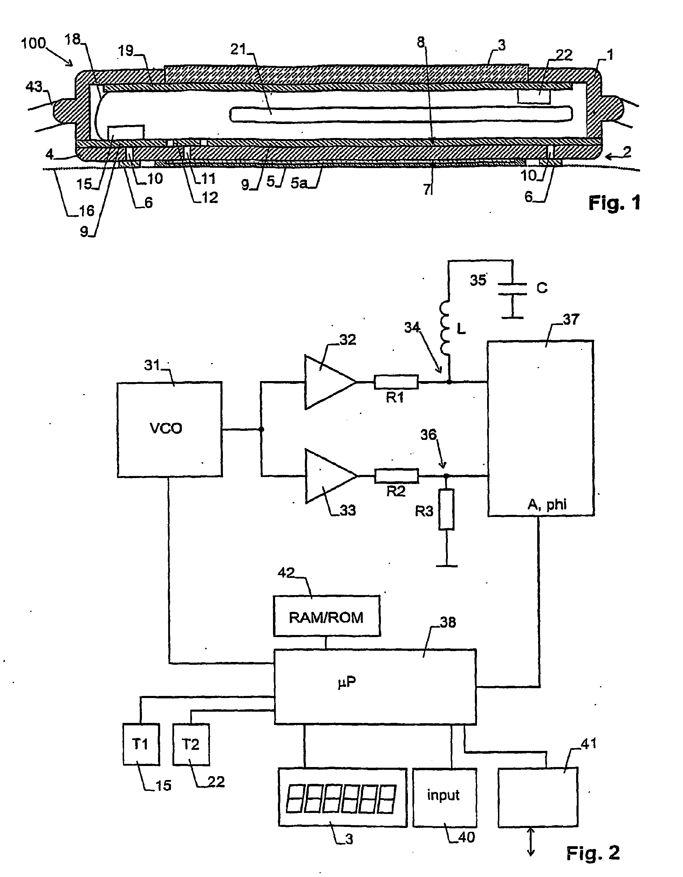

Fig. 1 shows a cross section of a device 100 for measuring a patient's glucose level. It comprises a housing 1 closed on one side by an electrode plate 2. A display 3 is arranged opposite electrode plate 2. Electronic circuitry is arranged between electrode plate 2 and display 3.

Electrode plate 2 comprises an electrically insulating substrate 4. A strip electrode 5 covered by an insulating layer 5a and a ring electrode 6 are arranged on an outer side 7 of insulating substrate 4. An inner side 8 of insulating substrate 4 is covered by a ground electrode 9. A plurality of though-contacts 10 connect ring electrode 6 to ground electrode 9. A further through-contact 11 connects one end of strip electrode 5 to a contact pad 12 arranged on inner side 8.

A first temperature sensor 15 is mounted to ground electrode 9 in direct thermal contact thereto. The large number of through-contacts 10 ensures that ground electrode 9 follows the temperature of ring electrode 6 and therefore the temperature of the specimen, the surface of which is indicated by a dotted line 16, closely.

Leads 18 are provided to connect ground electrode 9, contact pad 12 and first temperature sensor 15 to the electronic circuitry arranged on a printed circuit board 19 forming an assembly of electronic components. Printed circuit board 19 is advantageously arranged on a side of the device that is substantially opposite to the side of electrode plate 2. A battery 21 for powering the circuitry is arranged between printed circuit board 19 and electrode plate 2.

A second temperature sensor 22 is arranged on printed circuit board 19 and in direct thermal contact thereto.

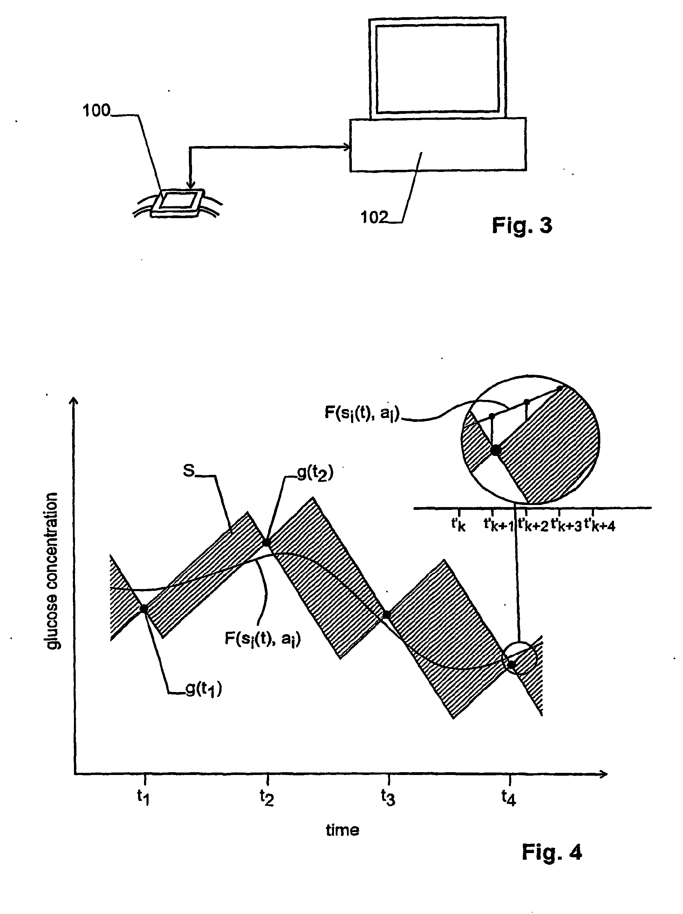

The design of the electrodes 5, 6, 9 of the present sensor can correspond to the one described in reference to Figs. 2 and 4 of WO 02/069791, which description is enclosed by reference herein.

Fig. 2 shows a block circuit diagram of the circuitry of device 100. It comprises a voltage controlled oscillator (VCO) 31 as a signal source for generating a sine wave signal or another periodic signal. This signal is fed to two amplifiers 32, 33. The output of first amplifier 32 is connected via a resistor R1 to a first signal path 34. A resonant circuit 35 comprising an inductance L and a capacitor C in series is connected between first signal path 34 and ground. The output of second amplifier 33 is connected via a resistor R2 to a second signal path 36. Second signal path 36 can be substantially identical to first signal path 34 but comprises a resistor R3 as a reference load instead of resonant circuit 35.

Both signal paths 34, 36 are fed to a measuring circuit 37, which determines the relative amplitude A of both signals and/or their mutual phase shift phi. Relative amplitude A can e.g. be the amplitude of first signal path 34 in units of the amplitude of second signal path 36 (wherein the amplitudes are the peak values of the sine waves).

The output signal of measuring circuit 37 is fed to a microprocessor 38, which also controls the operation of VCO 31.

Microprocessor 38 further samples the first and second temperature signals T1, T2 from first and second temperature sensors 15, 22. It also controls display device 3, an input device 40 with user operable controls, and an interface 41 to an external computer. A memory 42 is provided for storing calibration parameters, measurement results, further data as well as firmware for microprocessor 38. At least part of memory 42 is non-volatile.

Inductance L of the device of Fig. 2 can be generated by a coil and/or by the leads and electrodes of capacitor C. Its value is generally known with reasonable accuracy.

Capacitor C of the device of Fig. 2 is formed between strip electrode 5 and ring electrode 6 and is used for probing the specimen. For this purpose, the electrodes are arranged on the skin 16 of the patient as shown in Fig. 1.

For a good and permanent contact with the patient's skin, the device is advantageously worn on an arm or leg and provided with a suitable holder or wrist band 43.

The geometry of the electrodes is selected such that the electric field generated by them extends into the specimen and the body liquid to be measured. Advantageously, at least one of the electrodes of the capacitor is electrically insulated such that capacitor C is primarily a capacitive load, the capacitance and loss of which depend on the electrical properties (i.e. the response) of the specimen at the frequency of VCO 1.

In summary, the device shown in Figs. 1 and 2 comprises:

- an electrode arrangement or sensor arrangement comprising the electrodes 5 and 6 and
- processing circuitry including the elements 31 - 33, 37, 38 for measuring the response of the sensor arrangement or electrode arrangement to an electrical signal and deriving the glucose level therefrom.

In addition, it can comprise at least two temperature sensors 15, 22, the signals of which depend in different manner on the skin temperature of the body and on the environmental temperature. Both these temperatures can be taken into account when determining the glucose level.

Basic principle of operation:

The basic principle of operation of the device is described in WO 02/069791.

To measure the concentration of glucose in the body fluid of the patient, microprocessor 38 can e.g. initiate a measurement cycle consisting of a frequency sweep of VCO 1. The sweep should start at a frequency $f_{max}$ above the expected resonance frequency f0 of the resonant circuit 5 and extend to a frequency $f_{min}$ below resonance frequency f0 (or vice versa). Typical frequencies are given in WO 02/069791. During this sweep, the electrical properties of the two signal paths 34, 36 will vary in different manner. The amplitude determined by measuring circuit A will fall to a minimum A0 at a characteristic frequency f0, as described in WO 02/069791. At the same time or close thereto, phase shift phi crosses zero.

Microprocessor 38 measures A0 and/or f0 as input values describing the physiological state of the patient's tissue. In addition to the input values of A0 and/or f0, microprocessor 38 measures the temperature values T1 and T2 as further input values. Using suitable calibration data, the glucose level can be derived from these input values.

Such calibration data can be determined in straightforward manner using methods known to the person skilled in the art. In the following, however, some advantageous techniques are presented for the determination of the glucose level with the type of device described here as well as for its calibration.

In general, microprocessor 38 will use a formula of the type

$$g = F(s_1, s_2, \ldots s_N, a_0, a_1, \ldots a_M) \qquad (1)$$

for determining the glucose level g (or a parameter indicative thereof) from N measured input values $s_1, s_2, \ldots s_N$ (N > 0), where the function F has M+1 parameters $a_0, a_1, \ldots a_M$ (M $\geq$ 0), at least some of which have to be determined in suitable calibration experiments.

The measured input values $s_i$ are e.g. values directly or indirectly derived from the amplitude A0, the corresponding frequency f0, and the temperatures T1, T2. The input values can e.g. be the most recent values measured or they can be a time average or a median over a given number of recent measurements.

In an advantageous embodiment, the values $s_1$ = A0, $s_2$ = f0, $s_3$ = T1 and $s_4$ = T2 are used.

The function F can be empirical or it can be based at least partially on a model describing the physical nature of the mechanisms involved.

Under the approximation that the relation between the glucose level g and the measured values $s_i$ is linear, we have

$$g = a_0 + a_1 \cdot s_1 + a_2 \cdot s_2 + \ldots a_N \cdot s_N \qquad (2a)$$

with M = N.

Equation (2a) has the advantage of being linear in the input values $s_i$ as well as the parameters $a_j$, which

simplifies calibration as well as evaluation. More refined models can, however, be used as well.

Temperature compensation:

It is presently understood that electrical properties of the topmost skin layers and therefore of the signals A0 and f0 depend not only on the glucose level, but also on the temperature Ts of the skin and underlying tissue as well as on the temperature Te of the environment. This is at least in part due to the fact these properties depend on the amount of blood in the skin and underlying tissue, which in turn affects the temperature of the skin. Hence, it is advantageous to measure the skin temperature, a first approximation of which can be derived from the signal from temperature sensor T1.

However, the skin temperature is not only a function of the amount of blood in the skin and underlying tissue, but also of the environmental temperature Te. Hence, it is also advantageous to measure the environmental temperature, a first approximation of which can be derived from the signal from temperature sensor T2.

Hence, device 100 is advantageously equipped with at least two temperature sensors T1 and T2, the signals of which depend in different manner on the temperatures Ts and Te, such that a measurement of T1 and T2 is indicative of both temperatures Ts and Te. Hence, at least one of the input values $s_i$ should be derived from the signal of first temperature sensor 15 and at least another one of the input values $s_i$ should be derived from the signals of second temperature sensor 22.

Advantageously, one of the temperature sensors is closer to the electrodes 5, 6 (and therefore to the body of the patient) than the other sensor. For example, the first temperature sensor 15 is arranged at the same side of housing 1 as the electrodes 5, 6 and the second temperature sensor 22 at the opposite side.

The measured values may also depend on the temperature of the electronic circuits because the properties of voltage sources, A/D-converters and other circuitry are generally temperature dependent. Hence, it may also be advantageous to measure a temperature that is indicative of the circuit temperature Tc. In the present embodiment, this is especially true for temperature T2, i.e. by using the signal from second temperature sensor 22, changes of the circuit temperature Tc can be accounted for. However, an additional third temperature sensor for specifically measuring circuit temperature Tc may be provided as well.

Calibration:

In the following, advantageous methods for calibrating the device are described.

*a) Parameter Determination:*

A basic calibration of the device is required for each new patient.

In a first step of the basic calibration, the patient undergoes a calibration phase in which the glucose level is measured repetitively by an alternative method of measurement, e.g. by a conventional invasive technique, in order to obtain a series of K reference values $g(t_1)$, $g(t_2)$, ... $g(t_K)$ at times $t_1$ through $t_K$. In the same period, the input values $s_i$ are measured repetitively at L times $t'_1$ through $t'_L$, wherein L can be much larger than K. All measured values $s_i(t'_j)$ (i = 1 ... N, j = 1 ... L) are stored, e.g. in memory 42 of the device.

In order to derive accurate and meaningful parameters over a wide range of measurement conditions, the blood glucose level g as well as the environment temperature Te is varied during the calibration phase. For example, the environment temperature is varied over at least 5 ˚C, preferably at least 10 ˚C, e.g. by carrying out indoors and outdoors measurements, and the glucose level is varied by at least 100 mg/dl, e.g. by the patient having a snack and by delaying and/or reducing insulin.

The calibration phase can e.g. extend over two days and include at least 10 reference values per day. Several reference values should be recorded in the periods during which the glucose level and/or temperature are varied as described above in order to obtain a full record of these events.

Alternatively to or in addition to an intensive calibration phase of two days, an extensive calibration can be carried out during a period of e.g. 15 days that allows the device to "adapt" to a given user. During this extensive calibration phase, reference measurements will again be carried out, e.g. invasively, even though at less frequent intervals.

The data recorded during the calibration phase can be used for finding appropriate values for at least part of the parameters $a_i$. For this purpose, the values obtained by function F according to equation (1) are compared against the reference values $g(t_i)$ or against values derived therefrom, and those parameters $a_i$ are determined for which this comparison gives a closest match.

In a most simple approach, the parameters $a_i$ can be obtained from a conventional least-squares fitting algorithm. Suitable algorithms are known to a person skilled in the art and are e.g. described by Press, Teukolsky, Vetterling and Flannery in "Numerical Recipes in C", Cambridge University Press, 2nd edition, 1992, Chapter 15. For evaluating the function F at the times $t_1$ through $t_k$, only the input values $s_i$ at the times closest to $t_1$ through $t_k$ are required.

This simple approach, however, will only exploit part of the available information. In particular, it ignores the information obtained by the measurements of the input values $s_i(t'_j)$ at times t'j other than the times $t_1$ through $t_k$.

In an advanced approach, the reference values $g(t_i)$ are used to calculate a prediction (interpolation) of the actual glucose levels at times between the measurement times $t_1, \ldots t_k$, in particular at all times $t'_1 \ldots t'_L$. Then, the deviation of this prediction from the value of function F for the corresponding input values $s_i$ is calculated and the total deviation is minimized by varying the parameters $a_i$.

An empirical, semi-empirical or theoretical model of the variation of the glucose level in a body can be used for calculating the prediction (interpolation).

An advantageous model is based on the understanding that the rate of change of the glucose level is limited. For human beings, a typical maximum rate of increase is $\dot{g}_{incr} = 3.5$ mg·dl$^{-1}$·min$^{-1}$ and a typical maximum rate of decrease is $\dot{g}_{decr} = 4$ mg·dl$^{-1}$·min$^{-1}$ as well. This allows to predict a set S of possible glucose values for any time t between the times $t_1$ through $t_K$ as depicted in Fig. 4. S is the set of values delimited by lines of slope $\dot{g}_{incr}$ and $\dot{g}_{decr}$ extending from the measured points $g(t_i)$.

Taking this model into account, a possible calibration procedure is based on the following steps:

- Step 1: The patient undergoes the calibration phase as mentioned above in which the K reference values $g(t_i)$ and the L×N input values $s_j(t'_i)$ are measured and recorded.
- Step 2: Equation (1) is fitted to the measured reference values $g(t_1) \ldots g(t_K)$ by evaluating

$$f_i = F(s_1(t_i) \ldots s_N(t_i), a_0 \ldots a_M) \qquad (3)$$

at each time $t_i$ and comparing $f_i$ to $g(t_i)$. If the input values $s_j(t_i)$ at time $t_i$ are not known (because none of the $t'_k$ matches $t_i$ exactly, an estimate of the values $s_j(t_i)$ from measured input values $s_j(t'_k)$ for at least one $t'_k$ close to $t_i$ can be used. Then the parameters $a_1 \ldots a_M$ are varied to find a set of parameters where the total deviation between the values $f_i$ and $g(t_i)$ is at a minimum, e.g. by minimizing the sum of the squares of all $f_i$. This basic fitting process provides a set of starting values for the parameters $a_i$ in the following step 3.

- Step 3: The deviation of

$$F(t'_i) = F(s_1(t'_i) \ldots s_N(t'_i), a_0 \ldots a_M) \qquad (4)$$

for all times $t'_i$ from the prediction S at the corresponding times $t'_i$ is minimized by varying the parameters $a_i$. This can e.g. be achieved by defining, for each time $t'_i$, a predicted distribution $S(t'_i)$ of the glucose value and by calculating a deviation $d_i$ by comparing the predicted distribution $S(t'_i)$ with the value $F(t'_i)$. In the model of Fig. 4, a suitable deviation di can e.g. be defined as

$$d_i = \begin{cases} F(t'_i) - S_{max}(t'_i) & \text{if } F(t'_i) > S_{max}(t'_i) \\ S_{max}(t'_i) - F(t'_i) & \text{if } F(t'_i) < S_{min}(t'_i) \\ 0 & \text{otherwise} \end{cases} \qquad (5)$$

with $S_{min}(t'_i)$ and $S_{max}(t'_i)$ being the range of the set S of Fig. 4 at time $t'_i$, i.e.

$$S_{max}(t'_i) = \min(g(t_j) + \dot{g}_{incr} \cdot (t'_i - t_j), \\ g(t_{j+1}) + \dot{g}_{decr} \cdot (t_{j+1} - t'_i)) \qquad (6a)$$

and

$$S_{min}(t'_i) = \max(g(t_j) - \dot{g}_{decr} \cdot (t'_i - t_j),$$
$$g(t_{j+1}) - \dot{g}_{incr} \cdot (t_{j+1} - t'_i)) \quad (6b)$$

where $t_j$ is the closest of the times $t_1 \ldots t_K$ prior to $t'_i$.

The parameters $a_1 \ldots a_M$ can then e.g. be found by minimizing the value

$$D = \sum d_i \qquad (7)$$

numerically. Corresponding techniques are known to the person skilled in the art and e.g. described in Chapter 10 of the book "Numerical Recipes in C" cited above.

It must be noted that step 2 is optional if the starting values of step 3 are obtained by some different method, e.g. from typical values, or if step 3 uses an algorithm that does not require starting values for the parameters. Alternatively, step 3 can be omitted if the results from step 2 are to be used directly.

It must further be noted that equations (5) through (7) are advantageous examples but can be replaced by other suited definitions.

For example, instead of using a prediction S that gives a simple range, a prediction providing a probability density $S(g, t'_i)$ can be used, indicating the probability to observe a given glucose value g at time $t'_i$. Such a probability can e.g. be derived from an empirical or semi-empirical model that predicts how probable a given value of the glucose level is at time $t'_i$, given the reference values $g(tj)$. Apart from the reference values, a suitable model can e.g. take the physiological parameters of the patient (e.g. body weight) as well as events during the calibration phase (e.g. food intake, insulin administration etc.) into account for improving the accuracy of the prediction.

Equation (7) can also be replaced by any other suitable measure for the deviation of the function F from the prediction S. In particular if the probability of a certain deviation di is known, the formula for D should be defined in such a manner that its minimum coincides with the set of parameters having the highest statistical probability. For details, we refer to the book "Numerical Recipes in C" cited above.

Calibration is preferably carried out with a system as shown in Fig. 3, where an external computer 102 can be connected to the device 100 through interface 41. Computer 102 can instruct device 100 to start a calibration process, whereupon device 100 can be disconnected from the computer and be applied to the patient for carrying out above step 1. Then the reference values $g(t_i)$ are entered into computer 102, and the measured input values $s_j(t'_i)$ are transferred to computer 102 via interface 41. Above steps 2 and 3 are carried out in computer 102 and the resulting parameters $a_i$ are transferred back to device 100, which, after a final test of the performance of the calculated parameters $a_i$, is then ready for regular operation.

Even though the capabilities of computer 102 may be integrated directly into device 100, it is generally advantageous to use a separate computer system for the convenience of its use and its computational power.

*b) Shift correction during calibration:*

During the above basic calibration, movements of the patient or other events may cause device 100 to change its position in respect to the patient's body. Displacements of this type will usually lead to a change in signal that should be accounted for.

For taking such shifts into account, it is advantageous to introduce additional auxiliary parameters $a_{00}$, $a_{01}$, ... $a_{0P}$ during the above calibration steps. Assuming that $a_0$ is a purely additive parameter in function F (such as in the example of equation (2)), equations (3) and (4) above are replaced by

$$f_i = F(s_1(t_i) \ldots s_N(t_i), 0, a_1 \ldots a_M) +$$
$$a_{00} \cdot b_0(t_i) + \ldots + a_{0P} \cdot b_P(t_i) \qquad (3')$$

and

$$F(t'_i) = F(s_1(t'_i) \ldots s_N(t'_i), 0, a_1 \ldots a_M) +$$
$$a_{00} \cdot b_0(t'_i) + \ldots + a_{0P} \cdot b_P(t'_i), \qquad (4')$$

where the functions $b_i(t)$ are 0 unless the time t is in the range $\tau_i \ldots \tau_{i+1}$, where they are 1.

In other words, the additive parameter $a_0$ of function F is set to 0 (or, equivalently, another fixed value), and it is replaced by parameter $a_{00}$ in time interval $\tau_0 \ldots \tau_1$, by parameter $a_{01}$ in time interval $\tau_1 \ldots \tau_2$, etc.

The times $\tau_0$ and $\tau_P$ are the start and end times of the calibration phase and the other times $\tau_i$ are the times when a "shrift" of device 100 is detected during the calibration phase. Such a shift can e.g. be detected because at least one of the input values $s_i$ (such as the amplitude $A_0$ or frequency $f_0$) changes by more than a given threshold value $\Delta s_i$ during two consecutive measurements. Details on how to detect such "shrifts" are discussed in the section "shift correction during measurements" below.

By using equations (3') and (4') instead of (3) and (4), the parameters $a_{00} \ldots a_{0P}$ and $a_1 \ldots a_M$ can be determined using steps 2 and 3 described in the previous section. The parameters $a_1 \ldots a_M$ can then be used during normal operation of the device.

As to additive parameter $a_0$, that parameter can be roughly approximated to be the median or average of parameters $a_{00} \ldots a_{0P}$, but it is preferably determined from later recalibration measurements as described in section "Recalibration" below.

Instead of using additive parameters $a_{00} \ldots a_{0P}$, multiplicative parameters might be used for this kind of correction as well. In that case, equations (3') and (4') should be changed accordingly.

In more general terms, a compensation of "shifts" or displacements of device 100 during calibration can be achieved by replacing at least one of the parameters, e.g. $a_0$, in equations (3) and (4) by

$$\sum_{i=0}^{P} a_{0i} \cdot b_i(t), \qquad (8)$$

with $b_i(t)$ being 1 for $\tau_i < t < \tau_{i+1}$ and 0 otherwise. The parameters to be replaced in this way are those parameters that are most sensitive to shifts of the device.

In most cases, it will be sufficient to apply this technique to the one additive or one multiplicative parameter in F. (Definition: A parameter a is additive if function f(a, ...) can be re-written as $a + f'(\ldots)$ with f' being independent of a; a parameter a is multiplicative if function f(a, ...) can be re-written as $a \cdot f''(\ldots)$ with f'' being independent of a).

Normal operation:

After calibration of the device, all or at least most of the parameters $a_0 \ldots a_M$ are known. In a very simple device, such as described in WO 02/069791, all parameters can be determined completely during calibration and then equation (1) can be used for determining the glucose level from the measured input values $s_i(t)$ in regular operation.

In the following, however, some additional steps are described that allow to improve the accuracy of the device.

*a) Recalibration*

After the calibration steps described above, all parameters $a_i$ are known if it is assumed that no shift correction is necessary, i.e. if it is assumed that the device is being held at a fixed position on the patient's body.

If a shift of the device against the body is to be compensated for, at least one parameter, such as the additive or multiplicative parameter $a_0$, can only be determined inaccurately during calibration because the device may have been displaced during calibration or between calibration and regular measurement. In that case it is advantageous to carry out recalibration measurements during regular operation, e.g. once a day after affixing the device to the body.

A recalibration measurement consists, in a simple embodiment, of a single measurement of the glucose level $g(t_0)$ by conventional means. This glucose level is then entered into device 100 with a command to carry out recalibration.

When ordered to carry out a recalibration, microprocessor 38 finds the solution or optimum agreement of

$$g(t_0) = F(s_1(t_0) \ldots s_N(t_0), a_0, a_1, \ldots a_M) \quad (9)$$

by varying one of the parameters, usually the additive or multiplicative parameter $a_0$. The parameter found in this way is then used for following measurements.

For solving equation (9), the input values $s_1(t_0) \ldots s_N(t_0)$ may be derived from a single measurement at time to or from an average, median or interpolation value of several measurements around time to. Assuming that parameters $a_1$ to $a_M$ are known, parameter $a_0$ can then be calculated e.g. numerically by a root finding algorithm as known to the person skilled in the art.

A corresponding recalibration means can e.g. be implemented as a firmware program for microprocessor 38.

*b) Shift correction during normal operation*

As mentioned in the section "shift correction during calibration" above, a movement or "shift" of the device 100 in respect to the body may cause a change in measured signals. Even if all parameters are known from calibration or recalibration measurements as described above, such a shift may invalidate subsequent measurements.

To avoid this, microprocessor 38 of device 100 is advantageously programmed to detect such a shift. For this purpose, at least one signal value v(t) can be monitored, wherein the signal value v(t) is any value that is derived directly or indirectly from at least one of the input values $s_i(t)$ and that shows a characteristic shift when device 100 is moved in respect to the patient's body.

In particular, the signal value v(t) can be one of the following:

- One of the input values $s_i(t)$; for example, frequency $f_0$ or amplitude $A_0$ can be used since both these values show a change when device 100 is moved.
- The glucose value g derived from function F in equation (1). This value also shows a change when the device is moved.
- Any intermediate result generated during the evaluation of function F that shows a easily detected change when device 100 is moved.

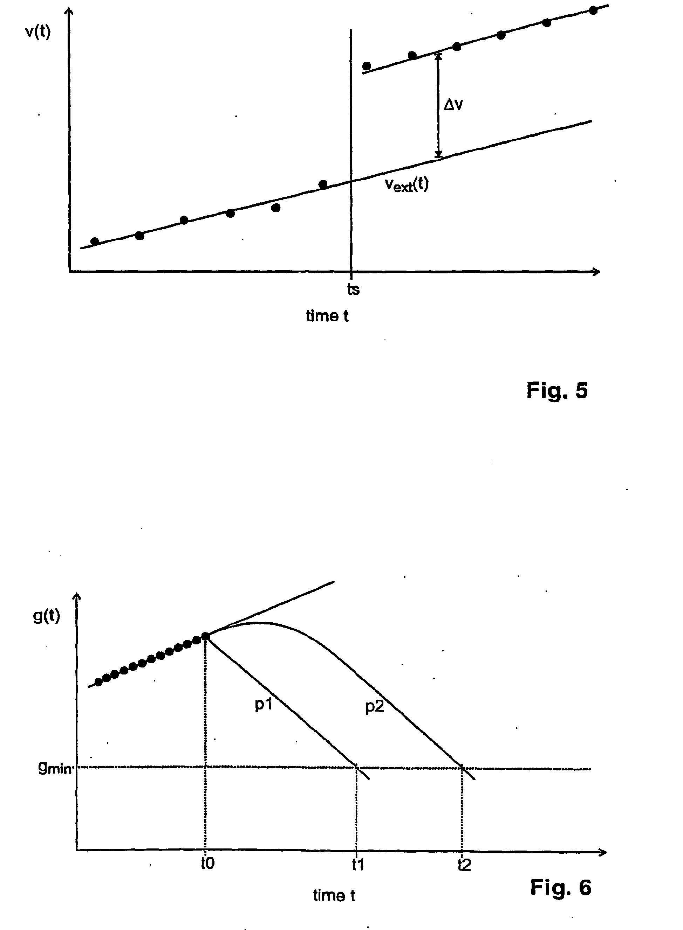

Fig. 5 shows a typical shift of signal value v(t) when device 100 is displaced along the patient's body at a time ts. As can be seen, prior and after the event, the signal value is fairly continuous (e.g. linear) while there is a sudden change between the measurements before and after time ts.

To detect a shift of this type, the following three steps are carried out at a given time t:

- Step 0: Calculate an extrapolated signal value $v_{ext}(t)$ as an extrapolation from a number of previous signal values v. Advantageously, $v_{ext}(t)$ is calculated only from signal values v older than t - $\Delta$t. $\Delta$t is a window length, which can e.g. be 5 minutes if one measurement is carried out each minute.
- Step 1: Determine an actual signal value $v_{act}(t)$ from one or more current signal values v. Advantageously, $v_{act}(t)$ is calculated from a median or average of the signal values within the time window t - $\Delta$t and t.
- Step 2: Compare the actual signal value $v_{act}(t)$ to the extrapolated signal value $v_{ext}(t)$ and assume that a "shift" has occurred if the values differ by a given threshold amount. This threshold should be larger than a typical noise-induced variation between consecutive signals and is e.g. in the order of 5% of a typical value of v(t) if v(t) = fo(t) is used. If the change exceeds the threshold amount, a shift correction procedure is started.

The shift correction procedure includes the following steps:

- Step 3: Define the exact time $t_s$ of the shift. This can e.g. be done by iterating over a given number of recent signal values v(t), e.g. the values in the above time window t - $\Delta$t and t, and looking for the largest change of consecutive values $v(t_i)$ and $v(t_{i-1})$.
- Step 4: Derive a shift correction $\Delta$v from an extrapolation of older values (e.g. the extrapolation $v_{ext}(t)$ mentioned above) and from values measured after the time $t_s$ of the shift. For example, the difference or ratio between

    - the median or average of the signal values in interval $t_s$ ... t and
    - the extrapolation $v_{ext}(t)$ can be calculated and be used as shift correction $\Delta$v. If the difference is used, the shift correction will be an additive correction to be added to v, otherwise it will be multiplicative correction to be multiplied to value v.

- Step 5: Use the shift correction for correcting subsequently measured glucose values. The specific implementation of this step depends on the definition of the signal value v(t). Examples:
- If signal value v(t) is equal to an input value $s_i(t)$, such as $f_0(t)$ or $A_0(t)$, subsequently measured input values should be corrected by $s_i(t) + \Delta v$ (additive correction) or by $s_i(t) \cdot \Delta w$ (multiplicative correction) before inserting them into function F for evaluation.
- If signal value v(t) is equal to the glucose value g(t) evaluated from function F, $\Delta v$ can be added to or multiplied with the returned function value. Alternatively, if F has an additive or multiplicative parameter $a_0$, that parameter can be corrected by addition of or multiplication with $\Delta v$.

Corrections for other types of signal values v(t) can also be implemented by correcting the input values, parameters, intermediate results or return value of function F in order to make sure that function F returns the same results before and after time $t_s$.

Above steps 0 to 5 can be implemented in a shift correction by suitable firmware in microprocessor 38 of device 100. In general, the shift correction should be able to

- detect a displacement of device 100 along the body of the patient, e.g. based on steps 0 to 2 above or any other method that is able to determine a sudden shift in a signal value,
- determine an effect of the shift on the measured glucose level, e.g. based on step 4 above, and
- correct the measured glucose levels after the shift to compensate for the determined effect.

It must be noted that the signal value used in steps 0 to 3 does not need to be the same as the one used in steps 4 and 5. It may be advantageous to use a raw.input signal, such as $f_0$ and $A_0$ for sensitively detecting a displacement of the device in steps 0 to 3, while it may be easier to carry out the correction on the function's F return value or an additive or multiplicative parameter $a_0$ in steps 4 and 5.

Range monitoring:

As mentioned above, an important purpose of device 100 is to provide a prediction of the time when a patient's glucose level may cross given safety limits.

For this purpose, microprocessor 38 comprises a software-implemented predictor that tries to predict when, at an earliest time, the glucose level g is likely to fall below a lower limit $g_{min}$ and/or to rise above an upper limit or $g_{max}$. Typical values for $g_{min}$ are in the order of 50 to 80 mg/dl, e.g. 70 mg/dl, and for $g_{max}$ they are above 160 mg/dl, e.g. 250 mg/dl.

Such predictors have been known to rely on the maximum rate of decrease is $\dot{g}_{decr}$ mentioned above, assuming that the first derivative g of the glucose level will never fall below the maximum rate of decrease, i.e. $\dot{g} \geq -\dot{g}_{decr}$ (if $\dot{g}_{decr}$ is defined to be a positive value).

It has been found, however, that this type of prediction can be improved. It has been found that not only the first derivative $\dot{g}$ of the glucose level is limited, but also the second derivative $\ddot{g}$. Typical lower and upper limits $\ddot{g}^{-}$ and $\ddot{g}^{+}$ were found to be both at 0.1 mg·d1$^{-1}$·min$^{-2}$.

This is illustrated in Fig. 6 showing a series of glucose level measurements g(t) indicated by dots. The lines p1 and p2 represent worst-case decay predictions starting from time t0. p1 is calculated on the mere assumption that $\dot{g} \geq -\dot{g}_{decr}$, while p2 is calculated from the refined assumption that $\dot{g} \geq -\dot{g}_{decr}$ and $\ddot{g} \geq -\ddot{g}^{-}$. As can be seen, the time t1 where prediction p1 reaches $g_{min}$ is smaller than the time t2 where prediction p1 reaches $g_{min}$. Hence, using prediction p2 allows to avoid unnecessary alerts and allows a more precise prediction.

To make a prediction of type p2, it is necessary to use not only the actual values g(t0) of the glucose, but also a first derivative $\dot{g}(t0)$ thereof. In the example of Fig. 6, time t2 - t0 can e.g. be calculated from g(t0), $\dot{g}(t0)$, $\ddot{g}^{-}$ and $\dot{g}_{decr}$ using simple analysis.

Instead of calculating a time t2 where a worst-case prediction g(t) is expected to reach $g_{min}$, it is also possible to make a worst-case prediction at a time t + $\Delta t$, where $\Delta t$ is a fixed "safety margin" of e.g. 20 minutes, and to compare this worst-case prediction g(t + $\Delta t$) e.g. to the lower threshold value $g_{min}$. If the worst-case prediction is below the threshold value, an alert is issued.

In general, range monitoring will therefore advantageously calculate a prediction of the glucose level from an estimate of the current value of the glucose level g(t0) as well as its derivative $\dot{g}(t0)$, taking into account that the prediction must fulfil the conditions $\dot{g} \geq -\dot{g}_{decr}$ and $\ddot{g} \geq -\ddot{g}^{-}$ and/or $\dot{g} \leq \dot{g}_{incr}$ and $\ddot{g} \leq \ddot{g}^{+}$

This type of monitoring can be used in the device 100 but also in any other type of device that has a detector for repetitively measuring the glucose level of a loving body. The prediction can, in particular, be used to provide an alert if the worst-case time until a hypoglycemia (g(t) < $g_{min}$) or hyperglycemia (g(t) > $g_{max}$) is below a given threshold time.

Remarks:

As it will be clear to the person skilled in the art, the methods described above can also be carried out with devices different from the one of Figs. 1 and 2, such as any of the devices shown in WO 02/069791 (taking into account that the temperature compensation described above will require the addition of a second temperature sensor).
Most aspects of the present invention, such as a temperature compensation, shift correction and various calibration methods, also work with devices using other types of sensors, such as optical sensors or inductive sensors.
While there are shown and described presently preferred embodiments of the invention, it is to be distinctly understood that the invention is not limited thereto but may be otherwise variously embodied and practiced within the scope of the following claims.

**Claims**

1.   A device for measuring a glucose level in a living body, comprising
   a detector comprising processing circuitry (31 - 33, 37, 38) for measuring the glucose level g(t) repetitively,
   a predictor for predicting a glucose level, wherein said predictor is designed for calculating a prediction of the glucose level from an estimate of the current value of the glucose level g(t) as well as its derivative $\dot{g}$(t),
   **characterized in that**, to calculate when, at an earliest time, the glucose level is likely to fall below a lower limit ($g_{min}$) or to rise above an upper limit ($g_{max}$) and to issue an alert if the earliest time is less than a given threshold time, said predictor is adapted to take into account that the prediction must fulfill the conditions

$$\dot{g} \geq -\dot{g}_{decr} \quad \text{and} \quad \ddot{g} \geq -\ddot{g}^{-}$$

   and/or

$$\dot{g} \leq \dot{g}_{incr} \quad \text{and} \quad \ddot{g} \leq \ddot{g}^{+}$$

   with $\dot{g}_{incr}$ being a maximum rate of increase, $\dot{g}_{decr}$ being a maximum rate of decrease and $\ddot{g}^{-}$ and $\ddot{g}^{+}$ being lower and upper limits for the second order derivate $\ddot{g}$ of the glucose level.

2.   The device of claim 1 comprising
   a sensor arrangement (5, 6) to be applied to a surface of the body,
   processing circuitry (31 - 33, 37, 38) for measuring a response of the sensor arrangement and deriving the glucose level therefrom, and
   at least a first and a second temperature sensor (15, 22) wherein a signal of the first temperature sensor depends in different manner on a skin temperature of the body and on an environmental temperature than a signal of the second sensor.

3.   The device of claim 2 wherein the first temperature sensor (15) is closer to said sensor arrangement (5, 6) than the second temperature sensor (22).

4.   The device of any of the claims 2 or 3 further comprising a housing (1) having a first side and a second side, wherein said sensor arrangement (5, 6) is arranged on said first side and wherein said first temperature sensor (15) is arranged at said first side and said second temperature sensor (22) at said second side.

5.   The device of any of the claims 2 to 4 wherein said first temperature sensor (15) is in thermal contact with said sensor arrangement.

6.   The device of any of the claims 2 to 5 further comprising an assembly (19) of electronic circuits wherein said second temperature sensor (22) is in thermal contact with said assembly (19).

7. The device (100, 102) of any of the preceding claims comprising
a sensor arrangement (5, 6) to be applied to a surface of the body,
processing circuitry (31 - 33, 37, 38) for measuring a response of the sensor arrangement and deriving the glucose level therefrom, wherein said processing circuitry (31 - 33, 37, 38) is adapted for calculating the glucose level g from

$$g = F(s_1, s_2, \ldots s_N, a_0, a_1, \ldots a_M),$$

where F is a function depending on $N \geq 1$ measured input values $s_1 \ldots s_N$, wherein the function F has M+1 calibration parameters $a_0 \ldots a_M$ with $M \geq 0$, and
calibration means (38, 102) for storing a series of input values $s_j(t'_i)$ recorded at times $t'_i$ in a given calibration phase and a series of reference values $g(t_i)$ measured at times $t_i$ in the calibration phase and deriving at least part of the parameters $a_i$ therefrom by comparing values obtained from the input values by function F against the reference values or against values derived from the reference values.

8. The device of claim 7 wherein said calibration means (102) is adapted for calculating at least part of said parameters $a_i$ by minimizing a deviation of the values

$$F(t'_i) = F(s_1(t'_i) \ldots s_N(t'_i), a_0 \ldots a_M)$$

from a prediction S of the glucose level at the times $t'_i$, wherein said prediction is derived from the reference values $g(t_i)$.

9. The device of claim 8 wherein said calibration means (102) is adapted to minimize the deviations

$$d_i = \begin{cases} F(t'_i) - S_{max}(t'_i) & \text{if } F(t'_i) > S_{max}(t'_i) \\ S_{max}(t'_i) - F(t'_i) & \text{if } F(t'_i) < S_{min}(t'_i) \\ 0 & \text{otherwise} \end{cases}$$

wherein $S_{min}(t'_i)$ and $S_{max}(t'_i)$ are minimum and maximum values of the glucose level at time $t'_i$.

10. The device of claim 9 wherein said calibration means (102) are adapted to minimize a sum of absolute values of the values $d_i$.

11. The device of any of the claims 7 to 10, wherein said calibration means (102) is adapted for
detecting the times $\tau_1 \ldots \tau_P$ when a shift of said device in respect to said body occurs during said calibration phase, and, for comparing values obtained by function F against the reference values $g(t_i)$ or against values derived from the reference values $g(t_i)$, replacing at least parameter $a_0$ of said parameters by

$$\sum_{i=0}^{P} a_{0i} \cdot b_i(t)$$

with $b_i(t)$ being 1 for $\tau_i < t < \tau_{i+1}$ and 0 otherwise, wherein $\tau_0$ and $\tau_{P+1}$ are the start and end times of the calibration phase.

12. The device of any of the claims 7 to 11 further comprising a recalibration means (38) for carrying out a recalibration step during which one of said parameters is varied to find an optimum agreement between the glucose level calculated from the function F and a glucose level from a reference measurement.

13. The device (100, 102) of any of the preceding claims comprising
a sensor arrangement (5, 6) to be applied to a surface of the body,
processing circuitry (31 - 33, 37, 38) for measuring a response of the sensor arrangement and deriving the glucose level therefrom, wherein said processing circuitry (31 - 33, 37, 38) is adapted for calculating the glucose level from

$$g = F(s_1, s_2, \ldots s_N, a_0, a_1, \ldots a_M),$$

where g is the glucose level and F is a function depending on $N \geq 1$ measured input values $s_1 \ldots s_N$, wherein the function F has M+1 calibration parameters $a_0 \ldots a_M$ with $M \geq 0$, and
a shift correction (38) adapted for detecting a displacement of said device in respect to said body, determining an effect of the shift on the measured glucose level and correcting the measured glucose level after the shift to compensate for the determined effect.

14. The device of claim 13 wherein said shift correction (38) is adapted for detecting the displacement by monitoring for a shift in a signal value v derived from at least one of the input values $s_i$.

15. The device of any of the claims 13 or 14 wherein said shift correction (38) is adapted to determine the effect of the shift on the measured glucose level by comparing an extrapolation ($v_{ext}(t)$) of signal values measured prior to the displacement with at least one signal value measured after the displacement.

16. The device of claim 15 wherein said shift correction (38) is adapted to determine the effect of the shift on the measured glucose level by calculating a difference between or a ratio of the extrapolation ($v_{ext}(t)$) and at the least one signal value measured after the displacement.

17. The device of any of the preceding claims wherein said processing circuitry (31 - 33, 37, 38) is adapted for calculating the glucose level g from

$$g = F(s_1, s_2, \ldots s_N, a_0, a_1, \ldots a_M),$$

where g is the glucose level and F is a function depending on $N \geq 1$ measured input values $s_1, s_2, \ldots s_N$, wherein the function F has M+1 calibration parameters $a_0, a_1, \ldots a_M$ with $M \geq 0$.

18. The device of claim 17 wherein parameter $a_0$ is an additive or multiplicative parameter in function F.

19. The device of any of the claims 17 or 18 wherein said processing circuitry (31 - 33, 37, 38) is adapted for calculating the glucose level g from $g = a_0 + a_1 \cdot s_1 + a_2 \cdot s_2 + \ldots a_N \cdot s_N$.

20. The device of any of the claims 17 to 18 wherein at least one of said measured input values is indicative of a response of the sensor arrangement (5, 6).

21. The device of any of the claims 17 to 20 further comprising
a signal source (31) for applying a frequency sweep to a signal path (34), wherein said sensor arrangement (5, 6) is connected to said signal path, and
a detector (37) for determining a characteristic frequency (f0) and/or amplitude (A0) at which a signal in said signal path (34) becomes minimum and/or a phase shift in said signal path goes through zero,
wherein said measured input values $s_1, s_2, \ldots s_N$ comprise a value indicative of said characteristic frequency (f0) and/or amplitude (A0).

22. The device of any of the claims 17 to 21 further comprising at least a first and a second temperature sensor (15, 22) wherein a signal of the first temperature sensor (15) depends in different manner on a skin temperature (Ts) of the body and on an environmental temperature (Te) than a signal of the second sensor (22), wherein said measured input values $s_1, s_2, \ldots s_N$ comprise signals (T1, T2) from said first and said second temperature sensors.

23. The device of any of the preceding claims comprising a holder (52) for affixing it to the body.

24. The device of any of the preceding claims wherein said sensor arrangement (5, 6) comprises an electrode arrangement with at least one electrode (5, 6), in particular at least two electrodes, and said processing circuitry comprises at least one signal source (31) for applying a signal to said electrode arrangement and a signal detector (37) for detecting a response from said electrode arrangement to said signal.

**25.** A method for predicting the glucose level in a living body comprising the steps of
measuring the glucose level g(t) repetitively,
predicting a future glucose level from an estimate of the current value of the glucose level g(t) as well as its derivative $\dot{g}(t)$,
**characterized in that** an earliest time when the glucose level is likely to fall below a lower limit ($g_{min}$) or to rise above an upper limit ($g_{max}$) is calculated and an alert if the earliest time is less than a given threshold time is issued by taking into account that the prediction must fulfill the conditions

$$\dot{g} \geq -\dot{g}_{decr} \text{ and } \ddot{g} \geq -\ddot{g}^-$$

and/or

$$\dot{g} \leq \dot{g}_{incr} \text{ and } \ddot{g} \leq \ddot{g}^+.$$

with $\dot{g}_{incr}$ being a maximum rate of increase, $\dot{g}_{decr}$ being a maximum rate of decrease and $\ddot{g}^-$ and $\ddot{g}^+$ being lower and upper limits for the second order derivate g of the glucose level.

**26.** The method of claim 25 comprising the steps of
applying a sensor arrangement (5, 6) to a surface of the body,
measuring a response of the sensor arrangement and deriving at least one first value (A0, f0) therefrom,
measuring at least a second and a third value (T1, T2) with a first and a second temperature sensor (15, 22), wherein the second value (T1) depends in different manner on a skin temperature of the body and on an environmental temperature than the third value (T2),
calculating from said first, second and third values (A0, f0; T1; T2) said glucose level using calibration parameters ($a_0$ ... $a_M$).

**27.** The method of any of the claims 25 or 26 wherein said glucose level g is determined by

$$g = F(s_1, s_2, \ldots s_N, a_0, a_1, \ldots a_M),$$

where F is a function depending on $N \geq 1$ measured input values $s_1, s_2, \ldots s_N$, wherein the function F has M+1 calibration parameters $a_0, a_1, \ldots a_M$;

**28.** The method of claim 27 wherein parameter $a_0$ is an additive of multiplicative parameter in function F.

**29.** The method of claim 28 wherein the glucose level g is calculated from $g = a_0 + a_1 \cdot s_1 + a_2 \cdot s_2 + \ldots a_N \cdot s_N$.

**30.** The method of claim 26 wherein said sensor arrangement (5, 6) comprises an electrode arrangement with at least one electrode (5, 6), in particular at least two electrodes, wherein a signal is applied to said electrode arrangement and a response from said electrode arrangement to said signal is measured.

**Patentansprüche**

**1.** Vorrichtung zur Messung eines Glukosespiegels in einem lebenden Körper, umfassend
einen Detektor umfassend Verarbeitungsschaltungen (31 - 33, 37, 38) zur wiederholten Messung des Glukosespiegels g(t),
einen Prädiktor zur Prognose eines Glukosespiegels, wobei der Prädiktor zur Berechnung einer Prognose des Glukosespiegels aus einer Schätzung des aktuellen Werts des Glukosespiegels g(t) sowie seiner Ableitung $\dot{g}(t)$ ausgestaltet ist,
**dadurch gekennzeichnet, dass** der Prädiktor für die Berechnung wann, zu einem frühesten Zeitpunkt, der Glukosespiegel unter einen unteren Grenzwert zu fallen ($g_{min}$) oder über einen oberen Grenzwert ($g_{max}$) zu steigen

scheint, derart ausgestaltet ist, dass er berücksichtigt, dass die Prognose die folgenden Bedingungen erfüllen muss und dass er eine Warnung ausgibt, wenn der früheste Zeitpunkt früher als ein gegebener Schwellenzeitpunkt ist:

$$\dot{g} \geq -\dot{g}_{decr} \text{ und } \ddot{g} \geq -\ddot{g}^-$$

und/oder

$$\dot{g} \leq \dot{g}_{incr} \text{ und } \ddot{g} \leq \ddot{g}^+$$

wobei $\dot{g}_{incr}$ eine maximale Steigungsrate, $\dot{g}_{decr}$ eine maximale Abnahmerate und $\ddot{g}^-$ und $\ddot{g}^+$ untere und obere Grenzen für die zweite Ableitung $\ddot{g}$ des Glukosespiegels sind.

2. Vorrichtung nach Anspruch 1, umfassend
eine Sensoranordnung (5, 6) zur Anwendung auf einer Oberfläche des Körpers,
Verarbeitungsschaltungen (31 - 33, 37, 38) zur Messung einer Antwort der Sensoranordnung und zur Ableitung des Glukosespiegels von der Messung, und
mindestens einen ersten und einen zweiten Temperatursensor (15, 22), wobei ein Signal des ersten Temperatursensors auf unterschiedliche Weise als der zweite Sensor von einer Hauttemperatur des Körpers und einer Umgebungstemperatur abhängt.

3. Vorrichtung nach Anspruch 2, wobei der erste Temperatursensor (15) näher an der Sensoranordnung (5, 6) als der zweite Temperatursensor (22) ist.

4. Vorrichtung nach einem der Ansprüche 2 oder 3, weiter umfassend ein Gehäuse (1) mit einer ersten und einer zweiten Seite, wobei die Sensoranordnung (5, 6) auf der ersten Seite angeordnet ist und wobei der erste Temperatursensor (15) auf der ersten Seite und der zweite Temperatursensor (22) auf der zweiten Seite angeordnet sind.

5. Vorrichtung nach einem der Ansprüche 2 bis 4, wobei der erste Temperatursensor (15) in thermischem Kontakt mit der Sensoranordnung ist.

6. Vorrichtung nach einem der Ansprüche 2 bis 5, weiter umfassend eine Anordnung (19) von elektronischen Schaltungen, wobei der zweite Temperatursensor (22) in thermischem Kontakt mit der Anordnung (19) ist.

7. Vorrichtung (100, 102) nach einem der vorangehenden Ansprüche, umfassend
eine Sensoranordnung (5, 6) zur Anwendung auf der Körperoberfläche,
Verarbeitungsschaltungen (31 - 33, 37, 38) zur Messung einer Antwort der Sensoranordnung und Ableitung des Glukosespiegels davon, wobei die Verarbeitungsschaltungen (31 - 33, 37, 38) zum Berechnen des Glukosespiegels g aus

$$g = F(s_1, s_2, \dots s_N, a_0, a_1, \dots a_M),$$

ausgestaltet ist, wobei F eine von $N \geq 1$ gemessenen Eingangswerten $s_1...s_N$ abhängige Funktion ist, wobei die Funktion F $M+1$ Kalibrationsparameter $a_0 ... a_M$ mit $M \geq 0$ hat, und
Kalibrationsmittel (38, 102) zum Speichern einer Reihe von Eingangswerten $s_j(t'_i)$ welche zu Zeitpunkten $t'_i$ während einer gegebenen Kalibrationsphase aufgenommen wurden und eine Reihe von Referenzwerten $g(t_i)$, welche zu Zeitpunkten $t_i$ während der Kalibrationsphase gemessen wurden und zum Ableiten mindestens eines Teils der Parameter $a_i$ davon, indem von den Eingangswerten mittels der Funktion F gewonnene Werte mit den Referenzwerten oder mit von den Referenzwerten abgeleiteten Werten verglichen werden.

8. Vorrichtung nach Anspruch 7, wobei das Kalibrationsmittel (102) zur Berechnung von mindestens einem Teil der Parameter $a_i$ durch Minimierung einer Abweichung der Werte

$$F(t'_i) = F(s_1(t'_i)...s_N(t'_i), a_0...a_M)$$

von einer Prognose S des Glukosespiegels zu Zeitpunkten $t'_i$ ausgestaltet ist, wobei die Prognose von den Referenzwerten $g(t_i)$ abgeleitet wird.

9. Vorrichtung nach Anspruch 8, wobei das Kalibrationsmittel (102) zur Minimierung der Abweichungen

$$d_i = \begin{cases} F(t'_i) - S_{max}(t'_i) & \text{wenn } F(t'_i) > S_{max}(t'_i) \\ S_{max}(t'_i) - F(t'_i) & \text{wenn } F(t'_i) < S_{min}(t'_i) \\ 0 & \text{anderenfalls} \end{cases}$$

ausgestaltet ist, wobei $S_{min}(t'_i)$ und $S_{max}(t'_i)$ minimale und maximale Werte des Glukosespiegels zum zeitpunkt $t'_i$ sind.

10. Vorrichtung nach Anspruch 9, wobei die Kalibrationsmittel (102) zur Minimierung einer Summe von Absolutwerten der Werte $d_i$ ausgestaltet sind.

11. Vorrichtung nach einem der Ansprüche 7 bis 10, wobei das Kalibrationsmittel (102) ausgestaltet ist zur
Detektion der Zeitpunkte $\tau_l...\tau_P$ wenn eine Verschiebung der Vorrichtung bezogen auf den Körper während der Kalibrationsphase stattfindet, und,
zum Vergleich von aus der Funktion F gewonnenen Werten mit den Referenzwerten $g(t_i)$, wobei mindestens der Parameter $a_0$ der Parameter durch

$$\sum_{i=0}^{P} a_{0i} \cdot b_i(t)$$

ausgetauscht wird, wobei $b_i(t)$ 1 ist für $\tau_i < t < \tau_i + 1$ und 0 anderenfalls, wobei $\tau_0$ und $\tau_{P+l}$ die Anfangs- und Endzeitpunkte der Kalibrationsphase sind.

12. Vorrichtung nach einem der Ansprüche 7 bis 11, weiter umfassend ein Rekalibrationsmittel (38) zur Durchführung eines Rekalibrationsschrittes während welchem einer der Parameter variiert wird, um eine optimale Übereinstimmung zwischen dem von der Funktion F berechneten Glukosespiegel und einem Glukosespiegel aus einer Referenzmessung zu finden.

13. Vorrichtung (100, 102) nach einem der vorangehenden Ansprüche, umfassend
eine Sensoranordnung (5, 6) zur Anwendung aud der Körperoberfläche,
Verarbeitungsschaltungen (31 - 33, 37, 38) zur Messung einer Antwort der Sensoranordnung und Ableitung des Glukosespiegels davon, wobei die Verarbeitungsschaltungen (31 - 33, 37, 38) zum Berechnen des Glukosespiegels g aus

$$g = F(s_1, s_2, ... s_N, a_0, a_1, ... a_M),$$

ausgestaltet ist, wobei g der Glukosespiegel und F eine von $N \geq 1$ gemessenen Eingangswerten $s_1...s_N$ abhängige Funktion sind, wobei die Funktion F M+1 Kalibrationsparameter $a_0 ... a_M$ mit $M \geq 0$ hat, und
eine Verschiebungskorrektur (38), welche zur Detektion einer Verschiebung der Vorrichtung bezogen auf den Körper ausgestaltet ist, wobei ein Effekt der Verschiebung auf den gemessenen Glukosespiegel bestimmt wird und der gemessene Glukosespiegel nach der Verschiebung zur Kompensation des bestimmten Effekts korrigiert wird.

14. Vorrichtung nach Anspruch 13, wobei die Verschiebungskorrektur (38) zur Detektion der Verschiebung durch Überwachung einer Verschiebung in einem Signalwert v, abgeleitet von mindestens einem der Eingangswerte $s_i$, ausgestaltet ist.

15. Vorrichtung nach einem der Ansprüche 13 oder 14, wobei die Verschiebungskorrektur (38) zur Detektion der Auswirkung der Verschiebung auf den gemessener. Glukosespiegel durch einen Vergleich der Extrapolation ($v_{ext}(t)$)

von Signalwerten, welche vor der Verschiebung gemessen wurden, mit mindestens einem nach der Verschiebung gemessenen Signalwert.

**16.** Vorrichtung nach Anspruch 15, wobei die Verschiebungskorrektur (38) zur Bestimmung der Auswirkung der Verschiebung auf den gemessenen Glukosespiegel durch Berechnung einer Differenz zwischen oder eines Verhältnisses der Extrapolation ($v_{ext}(t)$) und mindestens einem nach der Verschiebung gemessenen Signalwert.

**17.** Vorrichtung nach einem der vorangehenden Ansprüche, wobei die Verarbeitungsschaltungen (31 - 33, 37, 38) zum Berechnen des Glukosespiegels g aus

$$g = F(s_1, s_2, \ldots s_N, a_0, a_1, \ldots a_M),$$

ausgestaltet ist, wobei g der Glukosespiegel und F eine von $N \geq 1$ gemessenen Eingangswerten $s_1 \ldots s_N$ abhängige Funktion sind, wobei die Funktion F M+1 Kalibrationsparameter $a_0 \ldots a_M$ mit $M \geq 0$ hat.

**18.** Vorrichtung nach Anspruch 17, wobei der Parameter $a_0$ ein Additiv- oder Multiplikativparameter der Funktion F ist.

**19.** Vorrichtung nach einem der Ansprüche 17 oder 18, wobei die Verarbeitungsschaltungen (31 - 33, 37, 38) zum Berechnen des Glukosespiegels g aus

$$g = a_0 + a_1 \cdot s_1 + a_2 \cdot s_2 + \ldots a_N \cdot s_N$$

ausgestaltet ist.

**20.** Vorrichtung nach einem der Ansprüche 17 oder 18, wobei mindestens einer der gemessenen Eingangswerten auf eine Antwort der Sensoranordnung (5, 6) schliessen lässt.

**21.** Vorrichtung nach einem der Ansprüche 17 bis 20, weiter umfassend
eine Signalquelle (31) zur Generierung eines Frequenzsweeps an einem Signalpfad (34), wobei die Sensoranordnung (5, 6) an dem Signalpfad angeschlossen ist, und
einen Detektor (37) zur Bestimmung einer charakteristischen Frequenz ($f_0$) und/oder Amplitude ($A_0$) bei der ein Signal im Pfad (34) minimal wird und/oder eine Phasenverschiebung im Signalpfad durch Null geht,
wobei die gemessenen Eingangswerte $s_1$, $s_2$, $\ldots s_N$ einen Wert umfassen, welcher auf die charakteristische Frequenz ($f_0$) und/oder Amplitude ($A_0$) schliessen lässt.

**22.** Vorrichtung nach einem der Ansprüche 17 bis 21, weiter umfassend mindestens einen ersten und einen zweiten Temperatursensor (15, 22), wobei ein Signal des ersten Temperatursensors (15) auf unterschiedliche Weise als das Signal des zweiten Sensors (22) von einer Hauttemperatur (Ts) des Körpers und einer Umgebungstemperatur (Te) abhängt, wobei die gemessenen Eingangswerte $s_1$, $s_2$, $\ldots s_N$ Signale (T1, T2) vom ersten und vom zweiten Temperatursensor umfassen.

**23.** Vorrichtung nach einem der vorangehenden Ansprüche, umfassend eine Halterung (52) zur Fixierung am Körper.

**24.** Vorrichtung nach einem der vorangehenden Ansprüche, wobei die Sensoranordnung (5, 6) eine Elektrodenanordnung mit mindestens einer Elektrode (5, 6), insbesondere mindestens zwei Elektroden, umfasst, und die Verarbeitungssohaltung mindestens eine Signalquelle (31) zum Anlegen eines Signals an die Elektrodenanordnung und einen Signaldetektor (37) zur Detektion einer Antwort von der Elektrodenanordnung auf das Signal umfasst.

**25.** Verfahren zur Prädiktion des Glukosespiegels in einem lebenden Körper, umfassend die Schritte
wiederholtes Messen des Glukosespiegels $\dot{g}(t)$, voraussagen eines zukünftigen Glukosespiegels auf der Basis einer Schätzung des aktuellen Glukosespiegels g(t) sowie seiner Ableitung ġ(t),
**dadurch gekennzeichnet, dass** ein frühester Zeitpunkt in dem der Glukosespiegel unter einen unteren Grenzwert zu fallen ($g_{min}$) oder über einen oberen Grenzwert ($g_{max}$) zu steigen scheint berechnet wird, und eine Warnung ausgegeben wird, wenn der früheste Zeitpunkt früher als ein gegebener Schwellenzeitpunkt ist, unter Berücksichtigung dass die Prädiktion die folgenden Bedingungen erfüllen muss:

$$\dot{g} \geq -\dot{g}_{decr} \quad \text{und} \quad \ddot{g} \geq -\ddot{g}^-$$

und/oder

$$\dot{g} \leq \dot{g}_{incr} \quad \text{und} \quad \ddot{g} \leq \ddot{g}^+$$

wobei $\dot{g}_{incr}$ eine maximale Steigungsrate, $g_{decr}$ eine maximale Abnahmerate und $\ddot{g}^-$ und $\ddot{g}^+$ untere und obere Grenzen für die zweite Ableitung $\ddot{g}$ des Glukosespiegels sind.

**26.** Verfahren nach Anspruch 25, umfassend die Schritte
aufsetzen einer Sensoranordnung (5, 6) auf die Oberfläche des Körpers,
messen einer Antwort der Sensoranordnung und ableiten mindestens eines ersten Wertes (A0, f0) davon,
messen mindestens eines zweiten und eines dritten Wertes (T1, T2) mit einem ersten und einem zweiten Temperatursensor (15, 22), wobei der zweite Wert (T1) auf unterschiedliche Weise als der dritte Wert (T2) von einer Hauttemperatur des Körpers und einer Umgebungstemperatur abhängt,
berechnen des Glukosespiegels aus dem ersten, dem zweiten und dem dritten Wert ($A_0$, $f_0$; T1; T2) mit Hilfe von Kalibrationsparametern ($a_0 \dots a_M$).

**27.** Verfahren nach einem der Ansprüche 25 oder 26, wobei der Glukosespiegel g aus

$$g = F(s_1, s_2, \dots s_N, a_0, a_1, \dots a_M),$$

ausgestaltet ist, wobei g der Glukosespiegel und F eine von $N \geq 1$ gemessenen Eingangswerten $s_1 \dots s_N$ abhängige Funktion sind, wobei die Funktion F M+1 Kalibrationsparameter $a_0 \dots a_M$, bestimmt wird.

**28.** Verfahren nach Anspruch 27, wobei der Parameter $a_0$ ein Additivparameter oder ein Multiplikativparameter der Funktion F ist.

**29.** Verfahren nach Anspruch 28, wobei der Glukosespiegel g aus

$$g = a_0 + a_1 \cdot s_1 + a_2 \cdot s_2 + \dots a_N \cdot s_N$$

berechnet wird.

**30.** Verfahren nach Anspruch 26, wobei die Sensoranordnung (5, 6) eine Elektrodenanordnung mit mindestens einer Elektrode (5, 6), insbesondere mindestens zwei Elektroden, umfasst, wobei ein Signal an die Elektrodenanordnung angelegt wird und eine Antwort von der Elektrodenanordnung auf das Signal gemessen wird.

**Revendications**

**1.** Dispositif pour la mesure d'un niveau de glucose dans un corps vivant, comprenant
un détecteur comprenant des circuits de traitement (31 - 33, 37, 38) pour mesurer le niveau de glucose g(t) de manière répétitive,
un prédicteur pour estimer le niveau de glucose, le prédicteur étant adapté pour calculer une prédiction du niveau de glucose à partir d'une estimation de la valeur actuelle du niveau de glucose g(t) ainsi que sa dérivée g(t),
**caractérisé en ce que**, pour calculer quand, au plus tôt instant, le niveau de glucose semble à baisser sous une limite inférieure (gmin) ou monter au dessus d'une limite supérieure (gmax), ledit prédicteur est adapté à considérer le fait que la prédiction doit satisfaire les conditions suivantes et à émettre une alerte si le plus tôt instant est inférieur à un seuil de temps donné :

$$\dot{g} \geq -\dot{g}_{decr} \text{ et } \ddot{g} \geq -\ddot{g}^{-}$$

et/ou

$$\dot{g} \leq \dot{g}_{incr} \text{ et } \ddot{g} \leq \ddot{g}^{+}$$

$\dot{g}_{decr}$ étant le taux de baisse maximale et $\ddot{g}^{-}$ et $\ddot{g}^{+}$ étant les limites inférieures et supérieures pour la dérivée seconde $\ddot{g}$ du niveau de glucose.

2. Dispositif selon la revendication 1, comprenant
une assemblée des capteurs (5, 6) pour l'application sur une surface du corps,
des circuits de traitement (31 - 33, 37, 38) pour mesurer une réponse de l'assemblée des capteurs et pour déduire le niveau de glucose de la réponse, et
au moins un premier et un deuxième capteur de température (15, 22), le signal du premier capteur de température dépendant d'une température de peau du corps et d'une température ambiante d'une manière différente qu'un signal du deuxième capteur.

3. Dispositif selon la revendication 2, le premier capteur de température (15) étant plus proche de l'assemblée des capteurs (5, 6) que le deuxième capteur de température (22).

4. Dispositif selon l'une des revendications 2 ou 3, comprenant en outre une carcasse (1) avec une première face et une deuxième face, ladite assemblée des capteurs (5, 6) étant arrangée du côté de ladite première face et ledit premier capteur de température (15) étant arrangé du côté de ladite première face et ledit deuxième capteur de température (22) du côté de ladite deuxième face.

5. Dispositif selon l'une des revendications 2 à 4, le premier capteur de température (15) étant en contact thermique avec ladite assemblée des capteurs.

6. Dispositif selon l'une des revendications 2 à 5, comprenant en outre une assemblée (19) des circuits électroniques, ledit deuxième capteur de température (22) étant en contact thermique avec ladite assemblée (19).

7. Dispositif (100, 102) selon l'une des revendications précédentes, comprenant
une assemblée des capteurs (5, 6) pour l'application sur une surface du corps,
des circuits de traitement (31 - 33, 37, 38) pour mesurer une réponse de l'assemblée des capteurs et déduire le niveau de glucose de la réponse, lesdits circuits de traitement (31 - 33, 37, 38) étant adaptés pour calculer le niveau de glucose g à partir de

$$g = F(s_1, s_2, \ldots s_N, \bar{a}_0, \bar{a}_1, \ldots \bar{a}_M),$$

F étant une fonction dépendante de $N \geq 1$ valeurs d'entrée mesurées $s_1 \ldots s_N$, la fonction F ayant M+1 paramètres de calibration $a_0 \ldots a_M$ avec $M \geq 0$, et
des moyens de calibration (38, 102) pour sauvegarder une série de valeurs d'entrée $s_j(t'_i)$ enregistrées aux instants $t'_i$ dans une phase de calibration donnée et une série de valeurs de référence $g(t_i)$ mesurées aux instants $t_i$ dans la phase de calibration et déduire au moins une partie des paramètres $a_i$ de ces valeurs par comparer des valeurs obtenues à partir des valeurs d'entrée par la fonction F avec les valeurs de référence ou avec des valeurs déduites des valeurs de référence.

8. Dispositif selon la revendication 7, le moyen de calibration étant adapté pour le calcul d'au moins une partie desdits paramètres $a_i$ par minimiser une déviation des valeurs

$$F(t'_i) = F(s_1(t'_i) \ldots s_N(t'_i), a_0 \ldots a_M)$$

d'une prédiction S du niveau de glucose aux instants $t'_i$, ladite prédiction étant déduite des valeurs de référence $g(t_i)$.

**9.** Dispositif selon la revendication 8, ledit moyen de calibration (102) étant adapté pour minimiser les déviations

$$d_i = \begin{cases} F(t'_i) - S_{max}(t'_i) & \text{wenn } F(t'_i) > S_{max}(t'_i) \\ S_{max}(t'_i) - F(t'_i) & \text{wenn } F(t'_i) < S_{min}(t'_i) \\ 0 & \text{autrement} \end{cases}$$

$S_{min}(t'_i)$ et $S_{max}(t'_i)$ étant des valeurs minimales et maximales du niveau de glucose à l'instant $t'_i$.

**10.** Dispositif selon la revendication 9, les moyens de calibration (102) étant adaptés pour minimiser une somme de valeurs absolues des valeurs $d_i$.

**11.** Dispositif selon l'une des revendications 7 à 10, ledit moyen de calibration (102) étant adapté pour
détecter les instants $\tau_l...\tau_F$ quand un déplacement dudit dispositif par rapport audit corps survient pendant ladite phase de calibration, et, pour
comparer des valeurs obtenues par la fonction F avec les valeurs de référence $g(t_i)$ ou avec des valeurs déduites des valeurs de référence $g(t_i)$, remplaçant au moins le paramètre $a_0$ desdits paramètres par

$$\sum_{i=0}^{P} a_{0i} \cdot b_i(t)$$

$b_i(t)$ étant 1 pour $\tau_i < t < \tau_i + 1$ et 0 autrement, $\tau_0$ et $\tau_{P+1}$ étant les instants de commencement et fin de la phase de calibration.

**12.** Dispositif selon l'une des revendications 7 à 11, comprenant en outre un moyen de récalibration (38) pour effectuer un pas de récalibration pendant lequel un desdits paramètres sont variés afin de trouver un accord optimale entre le niveau de glucose calculé par la fonction F et un niveau de glucose d'une mesure de référence.

**13.** Dispositif (100, 102) selon l'une des revendications précédentes, comprenant
une assemblée des capteurs (5, 6) pour l'application sur une surface du corps,
des circuits de traitement (31 - 33, 37, 38) pour mesurer une réponse de l'assemblée des capteurs et déduire le niveau de glucose de la réponse, lesdits circuits de traitement (31 - 33, 37, 38) étant adaptés pour calculer le niveau de glucose g à partir de

$$g = F(s_1, s_2, ... s_N, a_0, a_1, ... a_M),$$

F étant une fonction dépendante de $N \geq 1$ valeurs d'entrée mesurées $s_1...s_N$, la fonction F ayant M+1 paramètres de calibration $a_0 ... a_M$ avec $M \geq 0$, et
une correction de déplacement (38) adaptée pour la détection d'un déplacement dudit dispositif par rapport audit corps, déterminant un effet du déplacement sur le niveau de glucose mesuré après le déplacement pour compenser l'effet déterminé.

**14.** Dispositif selon la revendication 13, la correction de déplacement (38) étant adaptée pour la détection du déplacement par surveiller un déplacement d'une valeur de signal v déduite d'au moins une des valeurs d'entrée $s_i$.

**15.** Dispositif selon l'une des revendications 13 ou 14, ladite correction de déplacement (38) étant adaptée pour déterminer l'effet du déplacement sur le niveau de glucose mesuré par comparaître une extrapolation ($v_{ext}(t)$) des valeurs de signal mesurées avant le déplacement avec au moins une valeur de signal mesurée après le déplacement.

**16.** Dispositif selon la revendication 15, ladite correction de déplacement (38) étant adaptée pour déterminer l'effet du déplacement sur le niveau de glucose mesuré par calculer une différence ou un rapport entre l'extrapolation ($v_{ext}(t)$) et au moins une valeur de signal mesurée après le déplacement.

**17.** Dispositif selon l'une des revendications précédentes, lesdits circuits de traitement (31 - 33, 37, 38) étant adaptés pour calculer le niveau de glucose g à partir de

$$g = F(s_1, s_2, \ldots s_N, a_0, a_1, \ldots a_M),$$

F étant une fonction dépendante de $N \geq 1$ valeurs d'entrée mesurées $s_1 \ldots s_N$, la fonction F ayant $M+1$ paramètres de calibration $a_0 \ldots a_M$ avec $M \geq 0$.

**18.** Dispositif selon la revendication 17, le paramètre $a_0$ étant un paramètre additif ou multiplicatif de la fonction F.

**19.** Dispositif selon l'une des revendications 17 ou 18, lesdits circuits de traitement (31 - 33, 37, 38) étant adaptés pour calculer le niveau de glucose g à partir de

$$g = a_0 + a_1 \cdot s_1 + a_2 \cdot s_2 + \ldots a_N \cdot s_N$$

**20.** Dispositif selon l'une des revendications 17 ou 18, au moins une desdites valeurs mesurées étant indicative d'une réponse de l'assemblée des capteurs (5, 6).

**21.** Dispositif selon l'une des revendications 17 à 20, comprenant en outre
une source de signal (31) pour appliquer un sweep de fréquence à un chemin de signal (34), l'assemblée des capteurs (5, 6) étant connectée au chemin de signal, et
un détecteur (37) pour la détection d'une fréquence caractéristique ($f_0$) et/ou amplitude ($A_0$) à laquelle un signal dans ledit chemin de signal (34) devient minimale et/ou un décalage de phase dans ledit signal passe à travers zéro, les valeurs d'entrée mesurées $s_1$, $s_2$, ... $s_N$ comprenant une valeur indicative de ladite fréquence caractéristique ($f_0$) et/ou amplitude ($A_0$).

**22.** Dispositif selon l'une des revendications 17 à 21, comprenant au moins un premier et un deuxième capteur de température (15, 22), le signal du premier capteur de température (15) dépendant de la température de peau (Ts) du corps et d'une température ambiante (Te) d'une manière différente qu'un signal du deuxième capteur (22), lesdites valeurs mesurées $s_1$, $s_2$, ...$s_N$ comprenant des signaux (T1, T2) dudit premier et deuxième capteur de température.

**23.** Dispositif selon l'une des revendications précédentes, comprenant un support (52) pour la fixation au corps.

**24.** Dispositif selon l'une des revendications précédentes, ladite assemblée des capteurs (5, 6) comprenant une assemblée d'électrodes avec au moins un électrode (5, 6), particulièrement au moins deux électrodes, et ledit circuit de traitement comprenant au moins une source de signal (31) pour l'application d'un signal à ladite assemblée d'électrodes et un détecteur de signal (37) pour la détection d'une réponse de ladite assemblée d'électrodes audit signal.

**25.** Méthode pour la prédiction du niveau de glucose dans un corps vivant, comprenant les pas de
mesurer le niveau de glucose g(t) de manière répétitive,
prédire un futur niveau de glucose d'une estimation de la valeur du niveau de glucose g(t) et de sa dérivée $\dot{g}$(t),
**caractérisée en ce qu'**un instant le plus tôt quand le niveau de glucose semble à baisser sous une limite inférieure ($g_{min}$) ou monter au dessus d'une limite supérieure ($g_{max}$) soit calculé et une alerte est émise si le plus tôt instant est inférieur à un seuil de temps donné par considérer le fait que la prédiction doit satisfaire les conditions

$$\dot{g} \geq -\dot{g}_{decr} \text{ et } \ddot{g} \geq -\ddot{g}^-$$

et/ou

$$\dot{g} \leq \dot{g}_{incr} \text{ et } \ddot{g} \leq \ddot{g}^+$$

$\dot{g}_{incr}$ étant un taux d'augmentation maximale, $g_{decr}$ étant un taux de baisse maximale et $\ddot{g}^-$ et $\ddot{g}^+$ étant des limites inférieures et supérieures pour la dérivée seconde $\ddot{g}$ du niveau de glucose.

**26.** Méthode selon la revendication 25, comprenant les pas de
appliquer une assemblé des capteurs sur la surface du corps,
mesurer une réponse de l'assemblé des capteurs et déduire au moins une première valeur (A0, f0) de la réponse,
mesurer au moins une deuxième et une troisième valeur (T1, T2) avec un premier et un deuxième capteur de température (15, 22), la deuxième valeur (T1) dépendant de la température de peau du corps et d'une température ambiante d'une manière différente que la troisième valeur (T2),
calculer à partir de la première, la deuxième et la troisième valeur ($A_0$, $f_0$; T1; T2) ledit niveau de glucose en utilisant des paramètres de calibration ($a_0$ ... $a_M$).

**27.** Méthode selon l'une des revendications 25 ou 26, ledit niveau de glucose g étant déterminé par

$$g = F(s_1, s_2, \dots s_N, a_0, a_1, \dots a_M),$$

F étant une fonction dépendante de $N \geq 1$ valeurs d'entrée mesurées $s_1...s_N$, la fonction F ayant M+1 paramètres de calibration $a_0$ ... $a_M$.

**28.** Méthode selon la revendication 27, le paramètre $a_0$ étant un paramètre additif ou multiplicatif de la fonction F.

**29.** Méthode selon la revendication 28, le niveau de glucose étant calculé à partir de

$$g = a_0 + a_1 \cdot s_1 + a_2 \cdot s_2 + \dots a_N \cdot s_N$$

**30.** Méthode selon la revendication 26, ladite assemblée des capteurs (5, 6) comprenant une assemblée d'électrodes avec au moins un électrode (5, 6), particulièrement au moins deux électrodes, un signal étant appliqué à ladite assemblé d'électrodes et une réponse de ladite assemblé d'électrodes audit signal étant mesurée.

**Fig. 1**

**Fig. 2**

**Fig. 3**

**Fig. 4**

**Fig. 5**

**Fig. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02069791 A

- US 20020106709 A

**Non-patent literature cited in the description**

- **PRESS ; TEUKOLSKY ; VETTERLING ; FLANNERY.** Numerical Recipes in C. Cambridge University Press, 1992